(19) Europäisches Patentamt
European Patent Office
Office européen des brevets

(11) **EP 3 672 483 B1**

(12) **EUROPEAN PATENT SPECIFICATION**

(45) Date of publication and mention
of the grant of the patent:
**26.05.2021 Bulletin 2021/21**

(51) Int Cl.:
*A61B 5/0507* (2021.01)          *A61B 5/00* (2006.01)
*A61B 5/053* (2021.01)

(21) Application number: **19723621.9**

(86) International application number:
**PCT/EP2019/061088**

(22) Date of filing: **30.04.2019**

(87) International publication number:
**WO 2020/088805 (07.05.2020 Gazette 2020/19)**

(54) **SYSTEM FOR DETECTION OF CANCERS IN HUMAN TISSUES**

SYSTEM ZUM NACHWEIS VON KREBS IN MENSCHLICHEM GEWEBE

SYSTÈME DE DÉTECTION DES CANCERS DANS LES TISSUS HUMAINS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.10.2018 EP 18203207**

(43) Date of publication of application:
**01.07.2020 Bulletin 2020/27**

(73) Proprietor: **Paegasus Médical SA**
**1227 Carouge GE (CH)**

(72) Inventor: **CANEPA, Stefano Nicolò**
**1227 Carouge (CH)**

(74) Representative: **Serravalle, Marco et al**
**Marco Serravalle S.L.**
**Residence Los Girasoles**
**Avenida Kurt Konrad Mayer 67, apt 16**
**38660 Adeje (ES)**

(56) References cited:
**EP-A1- 2 465 428          EP-A1- 3 257 439**
**US-A1- 2004 058 343       US-A1- 2008 045 832**
**US-A1- 2014 378 810       US-A1- 2017 007 149**
**US-A1- 2018 289 291       US-B1- 9 504 404**

**Description**

**Field of the invention**

. The present invention is directed to a system for recognition of biological alteration in tissues, in particular it relates to a system making use of electromagnetic waves with an operating frequency comprised between 2.3 and 2.5 GHz.

**Background of the invention**

. The use of radio frequencies for recognition of biological alteration in tissues has been disclosed in several patent applications. WO 01/07909 (Vedruccio) discloses an apparatus comprising a coherent transceiver probe which generates radiofrequencies at three different wavelengths (436, 872 and 1308 MHz) and a spectrum analyzer. The probe is manually swept along the body of the patient and the decrease or disappearance of one of the three peaks is considered as an indicator of a type of alteration. Because of this, the three different wavelengths are considered essential. The apparatus, commercially known as Trimprob, was used by many doctors, but considered scarcely reliable since the results are strongly dependent on the operator.

. EP 2 364 647 (Centro Studi e Ricerche Sant' Angela srl) discloses an apparatus for the detection of anomalies in biological tissues which tries to overcome the limits of the prior art. In particular, the apparatus addresses the problem of the lack of reliability by keeping the distance between transmitter and antenna constant. The apparatus of '647 tried also to overcome the dependency on the operator by moving the antenna automatically. The preferred frequency range is between 400 and 1000 MHz.

. EP 2 465 428 (Medielma srl) is also addressed to the identical problem and tries to solve it by increasing the number of receiving antennas so that one of the receiving antennas is always connected to the transmitter and, consequently, the apparatus is capable of detecting the anomalies. The apparatus operates at a frequency lower than 700 MHz. In fact, the patent considers very important to irradiate the tissue in "near-filed conditions". According to the patent, such condition can only be reached if the wavelength is lower than 700 MHz. Claim 1 of the granted patent is directed to a tissue anomaly detection system (1000), comprising: a probe antenna device (100) structured to radiate a narrowband incident radio-frequency signal at a frequency below 700 MHz to irradiate a tissue in near-field conditions so as to produce a resulting radio-frequency signal; a variable frequency oscillator (E-LS) that operates as an electromagnetic wave source connected to first and second modulating modules (MOD1-MOD2) to provide electromagnetic signals; a controller module (4) configured to control the a variable frequency oscillator (E-LS) that operates as electromagnetic wave source; a receiving device (200) structured to receive the resulting signal and provide corresponding received data; a processing module (300) structured to process the received data to provide an information indicating detection of an anomaly in said tissue; wherein: the probe antenna device has a fixed radiation frequency range and is structured so as that the resulting signal is a scattered signal resulting from a combination of the incident signal and an induced radio-frequency signal produced by the radiated tissue; the probe antenna device (100) includes at least one first transmitting antenna (1) connected to the first modulating module (MODI) and structured to radiate first electromagnetic waves having a first linear polarization and at least one second transmitting antenna (2) connected to the second modulating module (MOD2) and structured to radiate second electromagnetic waves having a second linear polarization orthogonal to the first polarization; the receiving device (200) includes at least one receiving antenna unit including two receiving antennas (R H1 -R V1) orientated to receive electromagnetic waves according to orthogonal linear polarisation and at least one demodulating module (DEM 11); the processing module (300) is structured to analyse from the resulting data an electrical field power scattering versus frequency pattern and detect said anomaly associated with nulls or minima in said pattern.

. EP 3 257 439 (Medielma srl) discloses an evolution of the apparatus of '428, wherein the antenna radiating element is rotated of a predetermined angle both in clockwise and counter clockwise direction by means of a servo mechanism. Also in this case, the attempt is to reduce human activity and define a standard method for performing the analysis.

. US 2004/0058343 discloses a method for detection of normal or abnormal constituents in cells, tissues or organisms, including proteins and nucleic acids, which method comprises obtaining a spectrographic waveform pattern of absorption or perturbation of electromagnetic radiation caused by a nucleic acid, protein, cell, tissue or organism suspected of having an abnormal condition and comparing the pattern with the spectrographic waveform pattern of the absorbance or perturbance of the same radiation by the normal or standard object.

. The results of the apparatuses according to the prior art, although useful, are considered as a preliminary screening, since the last word about the presence of a cancer is always left to other exams, e.g. NMR analysis colonoscopy, biopsy, etc.. However, the above exams are expensive and invasive.

. US 2017/007149 discloses an imaging system for detection of anomalies in the human body by the use of microwaves in the range from 10 to 26 GHz. The system is intended as a substitute of X-ray imaging and does not suggest the use of a microwaves in the frequency range from 2 to 3 GHz.

. Thus, it would be highly desirable to be able to reduce the number of patients undergoing the above invasive exams. The present invention represents a very important step in this direction and provides an analytical tool with a reliability which is comparable to the more expensive imaging examinations.

. The present invention is directed to a system for recognition of biological alteration in human tissues, more specifically for the detection of a cancer in some organs of the human body, without necessarily go for an imaging examination and it results in a diagnosis which is accurate, not only in terms of the correct assessment whether a cancer is present, but also about the location of the cancer.

## Summary of the invention

. The present invention is directed to a system for the detection of cancer, according to independent claim 1. The use of a frequency within the range 2.3-2.5 GHz allows a very reliable diagnosis and localization of the cancer.

## Brief description of the drawings

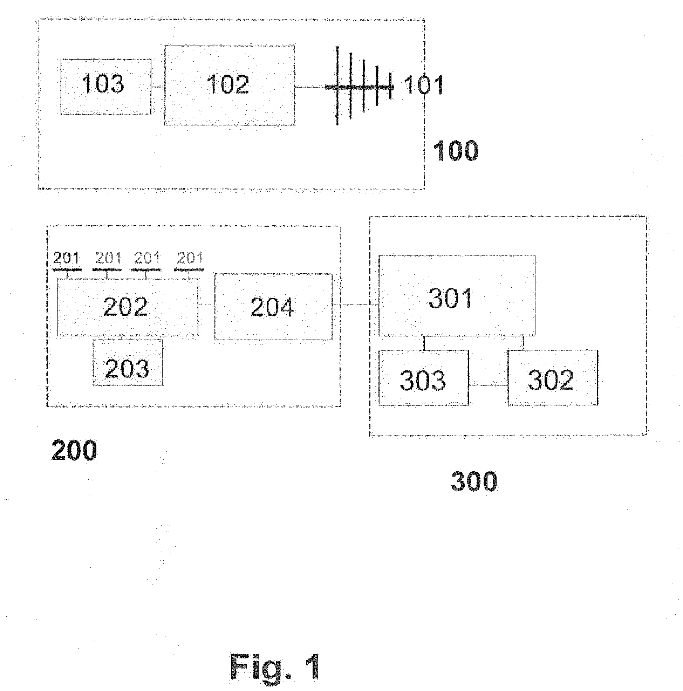

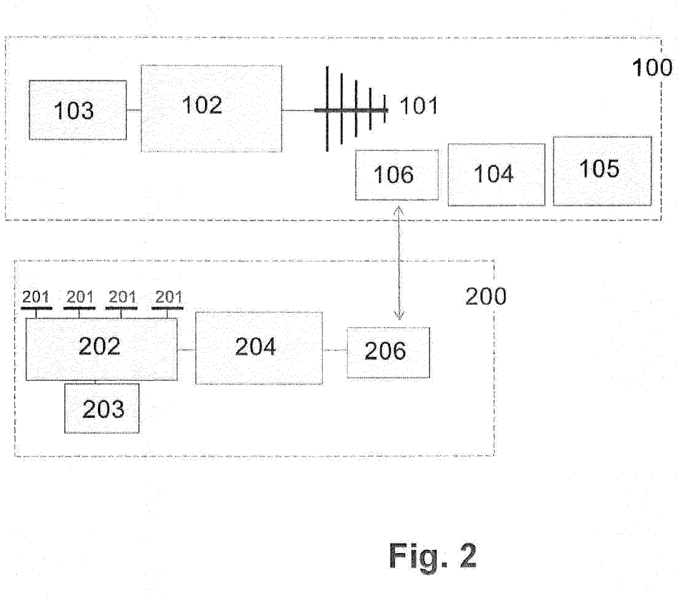

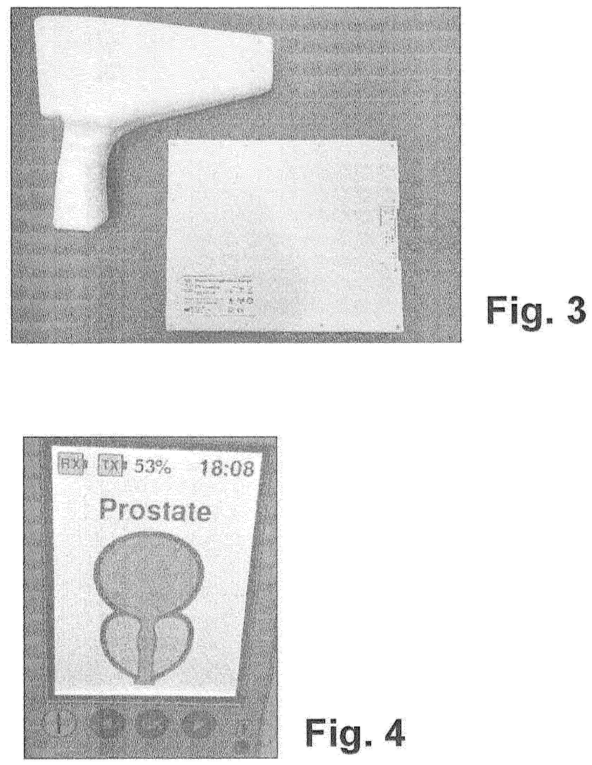

. Figure 1 is a block diagram of an embodiment of the device according to the invention.
. Figure 2 is a block diagram of another preferred embodiment of the invention.
. Figure 3 is a picture of a system according to the block diagram of figure 2.
. Figure 4 represents the screen of the transmitter of the system of figure 3.

## Detailed description of the invention

. The invention is directed to a system for the detection of cancer comprising: a transmitter device comprising at least one transmitting antenna, a transmitter, and a power supply; a receiving device comprising more than one receiving antenna, a receiver, a pre-processing module, and a power supply; and a data processing device comprising a microprocessor, a display and a power supply; wherein the transmitter device and the receiving device are configured to operate at a frequency comprised between 2.3 GHz and 2.5 GHz.

. The block diagram of Fig.1 shows a transmitter device 100 comprising an antenna 101, a transmitter 102, and a power supply 103; a receiver device 200, comprising four receiving antennas 201, a receiver 202, a pre-processing module 204, and a power supply 203; and a data processing device 300 comprising a microprocessor 301, a display 302 and a power supply 303.

. Fig.2 represents another preferred embodiment of the invention wherein the data processing device 300 is integrated in the transmitter device 100 together with a Bluetooth transmitter/receiver 106. In this embodiment, the transmitter 100 comprises an antenna 101, a transmitter 102, a power supply 103, a Bluetooth transmitter/receiver 106, a microprocessor 104 and a display 105. The receiving device 200 also comprises a Bluetooth transmitter 206 and sends the data to microprocessor 104 through Bluetooth transmitter/receiver 106. In this embodiment, the operator can see directly on transmitter 100 the result of the analysis.

. Figure 3 is a picture of a prototype of the system according to the block diagram of figure 2. The transmitter device comprises a screen which receives data from the receiver device through a Bluetooth connection. This prototype is very light and can be easily transported in a bag. The receiver is of a size of about 0.25 m x 0.20 m and contains four antennas positioned in a square configuration.

. Figure 4 shows the display of the transmitter of Figure 3. Form the display the operator can follow the response of the analysis.

. In one embodiment, the transmitter device comprises a single antenna, which is optionally a resonant cavity dipole antenna, or a directional antenna to be used in case of small organs, and a quartz oscillator to fix the specific emitted radio-frequency. Preferred types of directional antennas are Yagi antennas. Preferably, the directional antenna used in the transmitter of the present inventions irradiates with an angle comprised between 10° and 30°.

. The transmitter is preferably structured to radiate a narrowband incident microwave frequency signal to irradiate a patient tissue. To the purpose of the present application, narrowband signal means a signal with a bandwidth Bw which is small enough to use the assumption that a response as a result of the interaction with the body can be considered constant within the bandwidth Bw, provided that 1/Bw is below the relaxation times of the irradiated patient biological tissues (typical Bw>1KHz). The frequency radiated by the transmitter device is comprised in the range from 2.3 GHz to 2.5 GHz, most preferably around about 2.4 GHZ, which means from 2.35 GHz to 2.45 GHz.

. The receiver device comprises more than one receiving antenna, a pre-processing module and a power supply.

. The power supply preferably comprises rechargeable batteries. The receiving antenna(s) are tuned on the frequency of the transmitting antenna, i.e. from 2.3 GHz to 2.5 GHz, preferably around about 2.4 GHZ, which means from 2.35 GHz to 2.45 GHz. The receiving antennas can be either directional or omni directional; preferably, the receiving antennas are capable of covering 180°.

. The pre-processing module preferably comprises a filter used to clean the signal excluding any other frequency different from the emitted frequency in order to eliminate electromagnetic noise of the environment. Signals from the system of receiving antennas are digitalized through the receiver and sent to the pre-processing module. From the pre-processing unit, the signal is sent to the processing unit which is preferably either in the transmitting device, or a separate device e.g. a laptop computer or an equivalent console.

. The data processing unit through a processing algorithm provides parameters representing measures of the backscattered wave-field in term of different characteristics such as attenuation, polarization, resonances and interferences. Most preferably, the data processing unit is configured to run algorithms which determine the presence of nulls or minima (i.e. values below a threshold) in the received signal and associated with the presence of tissue anomalies, more specifically cancers. Examples of these algorithms are disclosed in EP 2 465 428. The data processing unit can also comprise a display and a power supply, which preferably comprises rechargeable batteries. As an alternative, the receiving device comprises a Bluetooth transmitter and communicates the data to transmitting device which comprises a data processing unit and visualizes the results of the diagnosis on a screen.

. The size of the receiver is compact. It is important to note that a receiver for the frequency of 2.4 GHz is small, since the size of an antenna is related to the wavelength $\lambda$ of the radiation (2.4 GHz, $\lambda$= 12 cm). Since more than one receiving antenna is used, the length of the receiver is preferably less than 0.80 m, more preferably less than 0.50 m even more preferably less than 0.30 m.

. The width of the receiver is preferably less than 0.30 m and the thickness less than 0.030 m. This reduced size and thickness of the receiver allows it to be placed on the medical couch under the patient in the position corresponding to the organ to be examined.

. In view of the reduced size of both the transmitter and the receiver, the device according to the invention can be easily transported to a patient bed or in a different location without any problem. In fact, the entire device can be placed in a bag, which allows easy transportation. The overall weight of the device according to the invention is very limited, e.g. lower than 10 kg, preferably lower than 5 kg, more preferably lower than 2 kg.

. The device according to the invention can be used in any room and on any available medical couch and does not require a dedicated medical couch and room.

. For this purpose, the device of the present invention is preferably provided with rechargeable batteries which allow operation of the device without the need of external power supply.

. Another advantage of the device of the present invention is the capability of detecting the position of the cancer. In the case of prostate cancer, the device according to the invention is capable of determining whether the cancer is located on the left or on the right lobe. To obtain this information, to date it was necessary to perform an NMR analysis. Analogously, for colorectal cancer, the device according to the invention allows a diagnosis of the intestinal tract where the cancer is present.

. The method of use of the device according to the invention is simple. The receiver is placed under the patient in correspondence to the organ to be checked. Then, the transmitter is turned on and placed in contact with the body as close as possible to the organ to be examined.

. In a preferred embodiment, the screen of the system of Fig. 3 and 4 will provide the operator with a screenshot requesting selection of the organ to be examined. A possible procedure for prostate and colon examination is herewith presented as a way of example. However, it is evident that a different presentation of information is possible. Similarly, other organs can be introduced in the menu of the system. If the organ is the prostate, the operator selects it and the screen will request examination of the left lobe of prostate. The operator will position the transmitter device in correspondence of the left lobe and will push a button on the screen to save the data. The screen will then request examination of the right lobe, and the operator will position the transmitter device on the right lobe and push the button again.

. When selecting colon as the organ, the screen will request analysis of 6 different positions, which correspond to the relevant points to be examined: 1) right iliac fossa (caecum and ascending colon), 2) right hip (ascending colon and hepatic flexure), 3) mesogastrium (transverse colon, 4) left hip (splenic flexure and descending colon), 5) left iliac fossa (descending and sigmoid colon), 6) hypogastrium (sigmoid and colorectal colon). The operator will position the transmitter on each position in sequence and push the button to acquire the data.

. Thus, in a preferred embodiment of the invention, the system comprises a software adapted to the examination of at least one of the following organs: prostate, colon, breast and thyroid. Preferably, the display of the system guides the examination and the operator by pushing a button saves the data measured by the system and indicating whether the examination is positive or negative. When all the area to be examined are completed the system provides an outcome of the examination (positive or negative) and possibly the area where the cancer is located (left or right lobe for prostate, the area of the colon for colon, etc.).

. Preferably, the processor is adapted to store data of a number of examinations corresponding to at least one working day. For example, the processor can store at least 100 results of examinations. In this manner, the operator can perform several examinations and at the end of the day connect the system to a processor such as a computer and download the data. Thus, the system of the invention is preferably adapted to transfer the stored data to an external processor. Transfer is preferably performed by the Bluetooth connection (106, 206).

**Experimental part**

**Prostate examination**

. 155 urologic patients were evaluated by multiparametric NMR, and by the system of figure 3 and 4 according to the invention (2.4 GHz). The results are confronted with the results of a prostate transrectal eco guided biopsy, which is considered the gold standard in prostate cancer diagnosis.

. For each methodology, two different analyses were performed: a first analysis on the entire prostate (Table 1), and a second analysis to assess the laterality of a possible prostate cancer (Table 2).

Table 1

|                       | 2.4 GHz | NMR |
| --------------------- | ------- | --- |
| True Positive (TP)    | 19      | 15  |
| False Positive (FN)   | 7       | 6   |
| True Negative (TN)    | 129     | 130 |
| False Negative (FN)   | 0       | 4   |

Table 2

|                       | 2.4 GHz | NMR |
| --------------------- | ------- | --- |
| True Positive (TP)    | 22      | 19  |
| False Positive (FN)   | 10      | 8   |
| True Negative (TN)    | 275     | 277 |
| False Negative (FN)   | 3       | 6   |

. The results on table 1 show that the device according to the invention did not produce any false negative and a number of false positive comparable to the number obtained by NMR. It is important to note that false negatives are the most dangerous cases, since when a false negative occurs, a person which is diagnosed sane, is in fact affected by a cancer. Tables 3 and 4 report a statistical evaluation of the data of table 1 and 2 respectively by calculating sensitivity, specificity, negative predictive value (NPV), positive predictive value (PPV), and accuracy, wherein:

.

$$Sensitivity = TP/(TP+FN) \cdot 100$$

.

$$Specificity = TN/(TN+FP) \cdot 100$$

.

$$PPV = TP/(TP+FP) \cdot 100$$

.

$$NPV = TN/(TN+FN) \cdot 100$$

.

$$Accuracy = (TP+TN)/(TP+FP+TN+FN) \cdot 100$$

Table 3 Statistical analysis of the results of Table 1

|  | Sensitivity | Specificity | PPV | NPV | Accuracy |
|---|---|---|---|---|---|
| 2.4 GHz | 100 | 94.85 | 73.08 | 100 | 95.48 |
| NMR | 78.95 | 95.59 | 71.43 | 97.01 | 93.55 |

Table 4 Statistical analysis of the results of Table 2

|  | Sensitivity | Specificity | PPV | NPV | Accuracy |
|---|---|---|---|---|---|
| 2.4 GHz | 88.00 | 96.49 | 68.75 | 98.92 | 95.81 |
| NMR | 76.00 | 97.19 | 70.37 | 97.88 | 95.45 |

. From the data of tables 3 and 4 it is evident that the device of the present invention is highly reliable and allows a screening of patients to detect the presence of a prostate cancer in an easy and economical manner. The device is also suitable for recognition of alteration of other organs, for example for the screening of colorectal cancer, breast cancer, thyroid cancer.

**Colon examination**

. It was performed a prospective single-center blinded study of consecutive adults undergoing colonoscopy. Before patients underwent colonoscopy, they were examined by the system according to the invention.

. During the procedure the subjects is dressed. A hand-held device was moved over the abdomen and electromagnetic response of tissues signals were recorded (2.4 GHz). A single investigator performed the test using the system. Abnormal signals were identified and recorded as malignant or benign (adenoma, hyperplastic polyps or diverticula). Findings were compared with those from colonoscopy. Statistical analysis was then performed.

RESULTS

. A total of 107 consecutive patients fulfilling the inclusion criteria were enrolled over a period of 5 months. The most frequent indication for colonoscopy was constipation, diarrhea, abdominal pain or fecal blood. The system according to the invention detected and characterized all 32 adenocarcinomas and polyps.

. The method identified cancers and polyps with 96,97% sensitivity, 78,38% specificity, and 84,11% diagnostic accuracy, compared to colonoscopy. The positive predictive value was 66,67% and the negative predictive value 98,31%. Among the 107 subjects, there were 16 false positive results (14,95%) and 1 false negative (0.93%) result. The results are summarized in Table 5.

Table 5

|  | Sensitivity | Specificity | PPV | NPV | Accuracy |
|---|---|---|---|---|---|
| 2.4 GHz | 96.97 | 78.38 | 66.67 | 98.31 | 84.11 |

. Colonoscopy is a very invasive examination which requires a long preparation, while examination by the system according to the invention is easy to perform and does not require any preparation.

**EP 3 672 483 B1**

**Claims**

1. System adapted to the detection of cancer, the system comprising:

   a. a transmitter device (100) structured to radiate a narrowband incident radiofrequency signal and irradiate a patient tissue, the transmitter device (100) comprising at least one transmitting antenna (101), a transmitter (102), and a power supply (103);
   b. a receiving device (200) comprising more than one receiving antenna (201), a receiver (202), a pre-processing module (204), and a power supply (203); and
   c. a data processing unit, configured to run algorithms which determine the presence of values below a threshold in the received signal associated with the presence of a cancer in the tissue under test, comprising a microprocessor (301; 104) and a display (302; 105);
   **characterized in that** the transmitter device (100) and the receiving device (200) are configured to operate at a frequency comprised between 2.3 GHz and 2.5 GHz.

2. System according to claim 1 wherein the data processing unit, comprising microprocessor (104) and display (105), is integrated in the transmitter device (100) together with a Bluetooth transmitter/receiver (106), and wherein the receiving device (200) also comprises a Bluetooth transmitter/receiver (206).

3. System according to claims 1-2, wherein the operating frequency is comprised between 2.35 GHz and 2.45 GHz.

4. System according to claims 1-3, wherein the transmitter device comprises a single antenna, and a quartz oscillator to fix the specific emitted radio-frequency.

5. System according to claims 1-4, wherein the transmitting antenna (101) is a directional antenna which irradiates with an angle comprised between 10° and 30°.

6. System according to claim 5, wherein the directional antenna is a Yagi antenna.

7. System according to claims 1-6, wherein the power supply (103, 203, 303) comprises a rechargeable battery.

8. System according to claims 1-7, wherein the processor comprises a software adapted to the examination of at least one of the following organs: prostate, colon, breast and thyroid.

9. System according to claims 1-8, wherein the microprocessor is adapted to store the results of a number of examinations.

10. The system of claim 9, wherein the microprocessor is adapted to store at least 100 results of examinations.

11. The system of claims 9-10, wherein the system is adapted to transfer the stored data to an external processor.


**Patentansprüche**

1. System, das für die Erkennung von Krebs geeignet ist, wobei das System aufweist:

   a. eine Sendevorrichtung (100), die zum Ausstrahlen eines schmalbandigen, einfallenden Hochfrequenzsignals und zur Bestrahlung eines Patientengewebes eingerichtet ist, wobei die Sendevorrichtung (100) mindestens eine Sendeantenne (101), einen Sender (102) und eine Stromversorgung (103) umfasst;
   b. eine Empfangsvorrichtung (200) mit mehr als einer Empfangsantenne (201), einem Empfänger (202), einem Vorverarbeitungsmodul (204) und einer Stromversorgung (203); und
   c. eine Datenverarbeitungseinheit, die eingerichtet ist, Algorithmen auszuführen, die das Vorhandensein von Werten unterhalb eines Schwellwerts in dem empfangenen Signal bestimmen, die mit dem Vorhandensein eines Krebses in dem zu testenden Gewebe verbunden sind, mit einem Mikroprozessor (301; 104) und einer Anzeige (302; 105),
   **dadurch gekennzeichnet, dass** die Sendevorrichtung (100) und die Empfangsvorrichtung (200) so ausgebildet sind, dass sie bei einer Frequenz zwischen 2,3 GHz und 2,5 GHz arbeiten.

**2.** System nach Anspruch 1, wobei die einen Mikroprozessor (104) und ein Display (105) aufweisende Datenverarbeitungseinheit zusammen mit einem Bluetooth-Sender/Empfänger (106) in die Sendevorrichtung (100) integriert ist, und wobei die Empfangsvorrichtung (200) ebenfalls einen Bluetooth-Sender/Empfänger (206) aufweist.

**3.** System nach einem der Ansprüche 1-2, wobei die Betriebsfrequenz zwischen 2,35 GHz und 2,45 GHz liegt.

**4.** System nach Anspruch 1-3, wobei die Sendevorrichtung eine einzelne Antenne und einen Quarzoszillator zur Fixierung der spezifischen ausgesendeten Funkfrequenz umfasst.

**5.** System nach einem der Ansprüche 1-4, wobei die Sendeantenne (101) eine Richtantenne ist, die mit einem Winkel zwischen 10° und 30° abstrahlt.

**6.** System nach Anspruch 5, wobei die Richtantenne eine Yagi-Antenne ist.

**7.** System nach einem der Ansprüche 1-6, wobei die Stromversorgung (103, 203, 303) eine wiederaufladbare Batterie umfasst.

**8.** System nach Anspruch 1-7, wobei der Prozessor eine Software umfasst, die zur Untersuchung von mindestens einem der folgenden Organe eingerichtet ist: Prostata, Kolon, Brust und Schilddrüse.

**9.** System nach Anspruch 1-8, wobei der Mikroprozessor dafür eingerichtet ist, die Ergebnisse einer Anzahl von Untersuchungen zu speichern.

**10.** System nach Anspruch 9, wobei der Mikroprozessor dafür eingerichtet ist, mindestens 100 Untersuchungsergebnisse zu speichern.

**11.** System nach Anspruch 9-10, wobei das System dazu eingerichtet ist, die gespeicherten Daten an einen externen Prozessor zu übertragen.


**Revendications**

**1.** Système adapté à la détection de cancer, le système comprenant :

a. un dispositif émetteur (100) structuré pour émettre un signal de radiofréquence incident à bande étroite et irradier un tissu de patient, le dispositif émetteur (100) comprenant au moins une antenne émettrice (101), un émetteur (102) et une alimentation (103) ;
b. un dispositif récepteur (200) comprenant plus d'une antenne réceptrice (201), un récepteur (202), un module de prétraitement (204) et une alimentation (203) ; et
c. une unité de traitement de données, configurée pour exécuter des algorithmes qui déterminent la présence de valeurs inférieures à un seuil dans le signal reçu associées à la présence d'un cancer dans le tissu sous test, comprenant un microprocesseur (301 ; 104) et un affichage (302 ; 105) ;
**caractérisé en ce que** le dispositif émetteur (100) et le dispositif récepteur (200) sont configurés pour fonctionner à une fréquence comprise entre 2,3 GHz et 2,5 GHz.

**2.** Système selon la revendication 1, dans lequel l'unité de traitement de données, comprenant un microprocesseur (104) et un affichage (105), est intégrée dans le dispositif émetteur (100) avec un émetteur/récepteur Bluetooth (106), et dans lequel le dispositif récepteur (200) comprend également un émetteur/récepteur Bluetooth (206).

**3.** Système selon les revendications 1 et 2, dans lequel la fréquence de fonctionnement est comprise entre 2,35 GHz et 2,45 GHz.

**4.** Système selon les revendications 1 à 3, dans lequel le dispositif émetteur comprend une seule antenne et un oscillateur à quartz pour fixer la radiofréquence émise spécifique.

**5.** Système selon les revendications 1 à 4, dans lequel l'antenne émettrice (101) est une antenne directionnelle qui irradie avec un angle compris entre 10° et 30°.

6. Système selon la revendication 5, dans lequel l'antenne directionnelle est une antenne Yagi.

7. Système selon les revendications 1 à 6, dans lequel l'alimentation (103, 203, 303) comprend une batterie rechargeable.

8. Système selon les revendications 1 à 7, dans lequel le processeur comprend un logiciel adapté à l'examen des organes suivants : la prostate et/ou le côlon et/ou le sein et/ou la thyroïde.

9. Système selon les revendications 1 à 8, dans lequel le microprocesseur est adapté pour stocker les résultats d'un certain nombre d'examens.

10. Système selon la revendication 9, dans lequel le microprocesseur est adapté pour stocker au moins 100 résultats d'examens.

11. Système selon les revendications 9 à 10, dans lequel le système est adapté pour transférer les données stockées vers un processeur externe.

**Fig. 1**

**Fig. 2**

Fig. 3

Fig. 4

**EP 3 672 483 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0107909 A
- EP 2364647 A
- EP 2465428 A
- EP 3257439 A
- US 20040058343 A
- US 2017007149 A